# EUROPEAN PATENT SPECIFICATION

(11) **EP 1 313 528 B1**
(45) Date of publication and mention of the grant of the patent: **25.04.2007**
(21) Application number: 01967645.1
(22) Date of filing: 30.08.2001
(51) Int. Cl.: A61M 37/00, A61F 9/007, A61M 25/06

(54) **MICROSURGICAL INJECTION AND/OR DISTENDING INSTRUMENTS AND SURGICAL APPARATUS UTILIZING SAME**
MIKROCHIRURGISCHE INJEKTION UND/ODER AUFWEITBARE INSTRUMENTE UND CHIRURGISCHE VORRICHTUNG ZU DEREN GEBRAUCH
INSTRUMENTS MICROCHIRURGICAUX D'INJECTION ET/OU DE DISTENSION ET APPAREIL CHIRURGICAL UTILISANT LESDITS INSTRUMENTS

(30) Priority: 31.08.2000 US 229051 P; 04.01.2001 US 753652
(43) Date of publication of application: 28.05.2003
(73) Proprietor: MPNB Ltd., Herzlia 46 733 (IL)
(72) Inventor: PAQUES, Michel, F-75012 Paris (FR); BENHAMOU, Nathanael, F-75017 Paris (FR)
(74) Representative: White, Duncan Rohan
(86) International application number: PCT/IL2001/000823
(87) International publication number: WO 2002/017986

(56) References cited:
- US-A- 5 257 979
- US-A- 5 364 374
- US-A- 5 417 209
- US-A- 5 616 118
- US-A- 5 695 479
- US-A- 5 725 514
- US-A- 5 893 837
- US-A- 6 024 719
- US-A- 6 036 678

## Description

### FIELD AND BACKGROUND OF THE INVENTION

The present invention relates to microsurgical instruments and apparatus utilizing such instruments. One embodiment of the invention is particularly useful for injecting a liquid or a suspension substance into a blood vessel in the retina of a subject's eye in order to treat certain eye diseases, such as retina disease, therein, and is therefore described below with respect to this application. Another embodiment of the invention is useful for catheterizing (or distending or cannulating) an occluded blood vessel, such as in a subject's eye, and is therefore described below also with respect to this application.

Venous occlusive diseases are among the most common retinal diseases seen in clinical practice. Recognition of these diseases is of particular importance because their complication may cause significant visual morbidity.

Central retinal vein occlusion (CRVO) is an acute occlusion of the central retinal vein of the eye and can lead to a severe decrease of vision. The exact mechanism of CRVO remains unknown, but there is strong evidence supporting that thrombus formation is the primary causative event. Many ocular and systemic conditions have been associated with CRVO, with glaucoma and systemic hypertension present in about 40% and 60% of the cases. The most common present complaint is an abrupt decrease in central vision. CRVO can also cause permanently damaging complications such as macular edema, one of the leading causes of visual loss in retinal pathology, and retinal ischemia, which can lead to irreversible loss of vision and neovascular glaucoma.

Branch retinal vein occlusion (BRVO) is an acute occlusion of one of the branch retinal veins, usually the temporal inferior or superior, and occurs almost exclusively at an arterio-venous intersection. The precise mechanism leading to a branch vein occlusion is still poorly understood, i.e., whether the occlusion is due to a thrombus, or to the compression of the artery on the retinal vein, or to both.

In both these conditions (CRVO and BRVO), the occlusion of the vein leads to a dramatic reduction of the vein retinal blood flow and thus of the drainage of the blood from the retinal circulation. The reduction of the blood flow is responsible for decrease of perfusion of the macular area and for macular edema and thus to a decrease of visual function.

Many treatments such as troxerutin, heparin, hemodilution, laser photocoagulation have been proposed, but none has proved to be effective, and none is used in current practice.

In order to restore the blood flow or to increase the drainage of the retinal blood, many procedures have been proposed: chorioretinal anastomosis induced by laser, intravenous fibrinolytic such as streptokinase or tPA.

However, it has been found that creating chorio-retinal anastomosis require high energy laser that can lead to unacceptable eyes complications such as choroidal and retinal neovascularization or vitreous hemorrhage. Moreover, a successful chorioretinal anastomosis is achieved in only a low percentage of the cases.

Treatment by injection of intravenous fibrinolytic such as streptokinase or RTPA has shown to be effective in CRVO. However, several complications such as hemiplegia or even fatal stroke have been described in those studies. Besides, according to a major cardiologic study (ISIS 3, Lancet 1992), the use of fibrinolytic is responsible for fatal stroke in about 0.5 % of the cases. Such risks inherent to injection of fibrinolytic in the general circulation are unacceptable for a non-life-threatening condition such as retinal vein occlusion.

In many organ systems, endovascular recanalization procedures such as percutaneous transluminal angioplasty and regional thrombolytic delivery have been effective in restoring blood flow. A recent study (Paques, Br J ophthalmol, 2000) suggested that infusion of urokinase into the ophthalmic artery through a microcatheter might improve the CRVO outcome in selected cases without death risk for the patient. However it remains a heavy procedure and the fibrinolytic agent is not delivered directly into the retinal vein.

These procedures even though they were not adopted as common therapies in CRVO, support the rationale of a direct approach to dissolve the thrombus. Indeed these procedures have shown that restoration of the vein retinal blood flow leads to a major improvement of the visual function.

Thus, we feel that increasing the bioavailability of the fibrinolytic molecule to the occlusion site in the retinal vein may improve the response to the treatment while lowering the side effects.

Accordingly, a device for introducing a fibrinolytic agent directly into the occluded blood vessel, at or near the site of the occlusion, and for catheterizing the occluded blood vessel with a miniaturized catheter is needed to disrupt the vein thrombus and to restore the retinal blood flow.
The cannulation of retinal vessels with glass micropipettes has already been described since 1987 (Allf, De Juan, Benner et al). The injection of a fibrinolytic agent in a retinal vein to treat CRVO in humans has been reported (Weiss JN. Ophthalmic Surg Lasers 2000 ;31 :162-16). For this procedure, the author used glass micropipettes and a manipulator.

Glass micropipettes are fragile and can easily be broken within the eye or within the retinal vein during the surgical procedure. This risk makes the procedure unsafe. Also, the external manipulator needed to stabilize the needle placed in the vessels in the XYZ axis makes the procedure cumbersome. Other prior art reflects numerous devices for ophthalmic surgery, including many devices for intraocular injections and/or illumination, as shown by the following USA Patents: Nos. 4,968,296; 5,201,730; 5,207,660; 5,364,374; 5,425,730; 5,725,514; 5,916,149; 5,843,071; 5,964,747; 6,004,302; 6,015,403. However, none of these known instruments appears to be suitable for the above treatment of venous occlusive diseases.

An object of the present invention is to provide a microsurgical instrument having a source of illumination therein, or usable with a microsurgical illumination instrument, particularly useful in the treatment of retinal diseases, particularly the above described ones. Another object of the invention is to provide a microsurgical injection instrument for injecting substances, particularly fibrinolytic agents, as well as other substances to be described below, into occluded blood vessels. A further object of the invention is to provide a microsurgical instrument that may be used for catheterizing blood vessels. A still further object of the invention is to provide a novel treatment for venous occlusive diseases.

US-A-5364374 discloses a device for microvascular injection comprising a reservoir attached to a blunted needle connected to a flexible tapered tubing. At the junction of the tubing and the needle is a spherical gripping means.

Document US 6, 024, 719 discloses an apparatus for performing surgery inside the human retina comprising a hollow microcannula having a distal end shaped to conform tangentially to the surface of the retina.

The present invention provides a microsurgical instrument as claimed in claim 1.

Preferably, the handpiece further comprises a stabilizer connected to or integrally formed with a distal portion of the hand-piece, the stabilizeer being positionale against the retina and serves for stabilization while penetrating the blood vessel in the subject's retina.

The mid part of the device placed within the eye can include a system that is used to obtain the coaxial placement of the needle and the vessel to be catheterized. This system will allow variation of angle "α" during the procedure.

Another improvement of the device includes a plate that is placed under the needle to be apposed at the surface of the retina, which plate is useful for stabilizing the needle by formation of contact between the retinal surface and the device during the penetration of a vessel by the miniaturized needle. Then, the plate located under the miniaturized needle at the distal end is apposed against the retina to improve the stability of the device during the penetration of the vessel.

As will be described more particularly below, such an instrument is particularly useful for the treatment of RVO by the injection of a fibrinolytic substance.

According to another aspect of the present invention, there is provided a microsurgical instrument comprising: handpiece having a proximal end graspable by the physician, and a distal end carrying a hollow needle sharpened at its tip for penetrating the blood vessel; the handpiece being formed with at least one passageway there through from its proximal end to the hollow needle at the distal end; and a flexible tube movable in the passageway through the hollow needle, after the needle has penetrated the blood vessel, to enter and catheterize the blood vessel.

As will be described below, such an instrument is particularly useful for the treatment of BRVO since it can also be used for distending or expanding the vein if the occlusion is caused partly or wholly by the compression of the vein.

In one described embodiment of the invention, the handpiece includes a second passageway there through from its proximal end to its distal end; and an optical fiber in the second passageway; the optical fiber having a distal end coaxial with the distal end of the handpiece and having an end face spaced from the hollow needle for illuminating the hollow needle and the blood vessel to be penetrated by the hollow needle.

Another embodiment is described, however, wherein the microsurgical injection instrument is used with a microsurgical illumination instrument also comprising a handpiece having a proximal end graspable by the physician, and a distal end to be inserted into the subject's eye; the illuminating instrument handpiece being formed with a passageway there through from the proximal end to the distal end; the latter passageway including an optical fiber having a proximal end to be exposed to a source of light, and a distal end to be located in the vicinity of the injection site in the subject's eye to illuminate same.

According to a further feature in the latter embodiment, the distal end of the handpiece of the microsurgical illumination instrument is constructed so as to be engageable with the distal end of the microsurgical injection instrument for stabilizing and guiding the distal end of the injection instrument when inserted into the subject's eye.

According to further features in yet another described embodiment, the instrument further includes an external guiding member for placement against the outer surface of the eye, and formed with a hole for receiving and guiding the hollow needle to penetrate the blood vessel in the subject's retina. The latter member is preferably made of a soft material, such as soft plastic, which controls the guided movement of the hollow needle.

According to a still further aspect of the invention, there is provided a method of treating a retinal venous occlusive disease in a subject comprising injecting a fibrinolytic agent into an occluded retinal vein of the subject by a microsurgical injection instrument including a handpiece having a proximal end graspable by the physician, and a distal end carrying a hollow needle sharpened at its tip for penetrating the blood vessel in the subject's retina.

According to yet another aspect of the invention, there is provided a method for treating an occluded blood vessel in a subject, comprising penetrating the occluded vein with a hollow needle having a sharpened tip, and moving a flexible tube through the hollow needle to catheterize the retinal vessel and disrupt the intraluminal thrombus.

Further features, advantages, and applications of the invention will be apparent from the description below.

### BRIEF DESCRIPTION OF THE DRAWINGS

The invention is herein described, by way of example only, with reference to the accompanying drawings, wherein:
Fig. 1 illustrates one form of microsurgical injection instrument constructed in accordance with the present invention;
Fig. 2 is a fragmentary view illustrating the instrument of Fig. 1 as used for catheterizing an occluded blood vessel to disrupt the intraluminal thrombus;
Fig. 3 illustrates one manner of using the microsurgical instrument of Fig. 1;
Fig. 4 illustrates the use of the microsurgical instrument of Fig. 1 together with a microsurgical illumination instrument in the treatment of a retinal disease;
Fig 5 is a side view diagrammatically illustrating the use of an external guiding member with the described instrument;
Fig. 6 is a front view of the external guiding member; and
Fig. 7 is a top view of the distal portion of the microsurgical injection instrument of the present invention, presenting, in partcicular a stabilizer positioned at a distal portion thereof.

### DESCRIPTION OF PREFERRED EMBODIMENTS

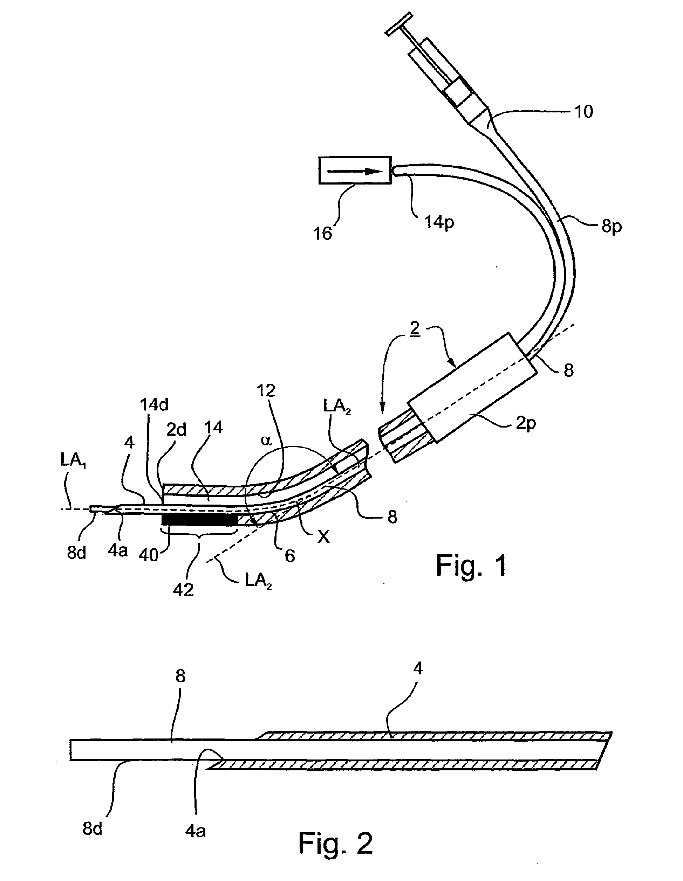

The microsurgical injection instrument illustrated in Fig. 1 is particularly useful by a physician for the treatment of retinal diseases, especially central retinal vein occlusion (CRVO) or branch retinal vein occlusion (BRVO), as briefly described above, by reestablishing retinal blood flow by pharmacological and mechanical means, by injecting a liquid substance or suspension, particularly a fibrinolytic agent into a blood vessel, and/or by catheterizing the blood vessel, in the retina of a subject's eye.

The illustrated instrument includes a handpiece, generally designated 2, of rigid material, plastic or metal. It has a finger-piece 2a at its proximal end 2p graspable by the physician, and a distal end 2d carrying a hollow needle 4 sharpened at its tip 4a for penetrating a blood vessel in the subject's retina. As will be described more particularly below, when the illustrated instrument is used for treating for CRVO or BRVO, the blood vessel penetrated would be a retinal vein, such as the central retinal vein; and the liquid substance injected into it would be a fibrinolytic agent to increase the bioavailability of the fibrinolytic molecule to the occlusion site.

In the instrument illustrated in Fig. 1, the handpiece 2 is formed with a passageway 6 therethrough from the proximal end 2p to the distal end 2d. When the instrument is to be used for injecting a substance, passageway 6 is used for delivering the substance to be injected by the needle 4 from a syringe 10 at the proximal end of the handpiece. When the instrument is to be used for catheterizing a blood vessel, passageway 6 receives a flexible tube, which is movable through the sharpened tip 4a of the hollow needle 4, after penetrating the blood vessel, to enter and catheterize the vessel and thereby to restore the blood channel.

The illustrated instrument includes a second passageway 12 for receiving an optical fiber 14 having its proximal end 14p exposed to a light source 16, for delivering the light via its distal end 14d to illuminate the needle 4 -and the needle insertion site during the venous puncture.

As shown in Fig. 1, the outer face of the distal end 14d of the optical fiber 14 is substantially-flush with the outer face of the distal end 2d of the handpiece 2, whereas the needle 4 projects outwardly from both faces. Such an arrangement better enables the optical fiber 14 to illuminate the needle 4 and the injection site at the time of the injection operation.

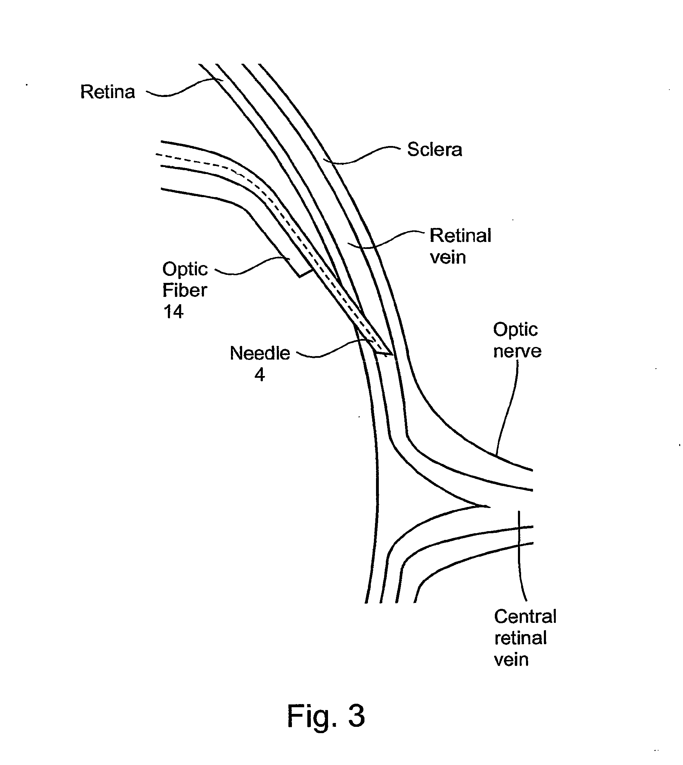

As shown in particularly in Fig. 1, the distal end 2d of the handpiece 2 is angulated at site "X" to an angle "α" to the proximal end 2p of the handpiece. Since the needle 4 is coaxial with the distal end 2d of the handpiece, the longitudinal axis LA₁ of the needle 4 is located at the same angle "α" with respect to the longitudinal axis LA₂ of the proximal end 2p of the handle 2. Angle "α" is from 90°-180° °, preferably from 120°-170°, most preferably about 145°. Angle "α" is preferably variable between 90° to 180° and could be modified during the procedure so that the distal end of the device, e.g., the miniaturized needle, will be parallel (coaxial) to the vessel to be penetrated. As will be described below particularly with respect to Fig. 3, this angulation of the distal end 2d of the handpiece with respect to the proximal end 2p orients the needle 4 substantially tangentially to the plane of the subject's retina, and thereby facilitates the penetration of the needle into the vein of the subject's retina.

The flexible tube 8 disposed within passageway 6 of the handpiece 2 is made of a soft, flexible material (nylon or soft silicon, for example) having a distal end 8d movable within the passageway through the hollow needle 4. As shown in Fig. 2, it has an outer diameter equal or inferior to the inner diameter of needle 4 so that the distal end 8d of the tube may be moved through the needle (as shown in full lines in Fig. 2 and in broken lines in Fig. 1) after the needle has penetrated the blood vessel, to enter and distend the blood vessel.

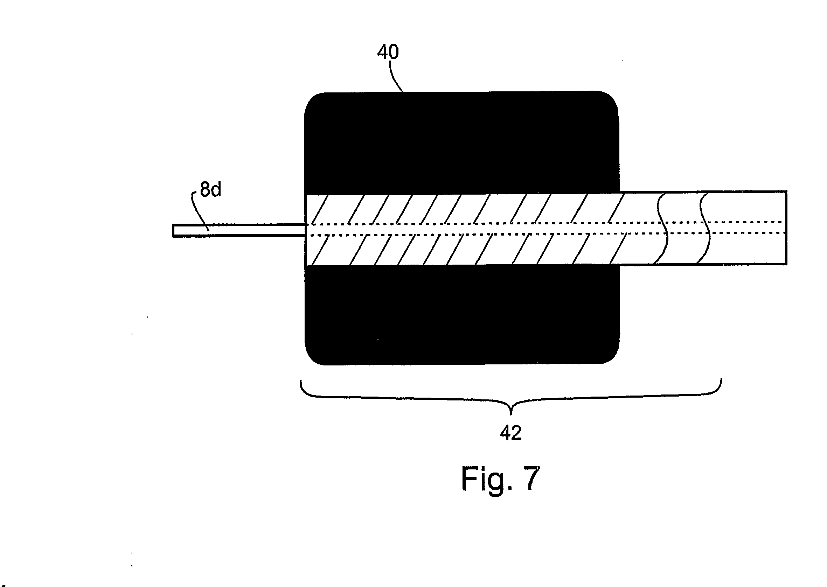

As illustrated in Figs. 1 and 7, the handpiece preferably further comprises a stabilizer 40, preferably shaped as a stabilizing plate, connected to or integrally formed with a distal portion 42 of the hand-piece. In use, stabilizeer 40 is positionale against the retina of the patient during the microinjection surgical procedure and serves for stabilization of the needle while penetrating a blood vessel in the subject's retina.

The instrument illustrated in Figs. 1-3 may be used to treat an occlusion, such as an acute occlusion, of the central retinal vein of the eye (CRVO) in the following manner as shown particularly in Fig. 3:

Under either local or general anesthesia, a conventional pars-plana approach with vitrectomy with separated infusion is performed. The instrument of Figs. 1 - 3 is used to introduce the needle 4 and the distal end 14d of the optical fiber 14 into the eye through a sclerotomy. The distal extremity of the needle is brought coaxially close to the retinal vein, approximately 500-2000 microns from the optic disc. The site of penetration of the vein can be nasal, temporal, inferior or superior according to the clinical and anatomical features of the fundus vessels of the eye to be treated. The retinal vein is then penetrated with the sharp distal end of the needle 4 (Fig. 3), which is preferably 30-120 microns diameter. A fibrinolytic agent, such as recombinant tissue plasminogen activator (rTPA) or streptokinase, is then injected to dissolve the vein thrombus.

The following procedure mightor not be associated to the first procedure:

After the fibrinolytic agent has been injected into the retinal vein, tube 8 is extended through the needle to enter the vein in order to catheterize the central retinal vein, to disrupt the central retinal vein thrombus and to recanalize the central retinal vein. At the end of the procedure, the needle and the tube are removed from the retinal vessel and then from the eye.

The illustrated instrument may also be used to treat BRVO in the following manner:

Under either local or general anesthesia, a conventional pars-plana approach and vitrectomy is performed. The retinal vein is penetrated upstream from the occlusion site, as close as possible from it (preferably 500 microns), by the sharp end of the hollow needle 4, which is preferably 30-120 microns diameter. This is done by bringing the sharp end of the needle close to the site of the occlusion (arterio-venous intersection), penetrating the vein, and injecting the fibrinolytic agent into the vein. After the fibrinolytic agent has been injected, tube 8 is extended through the needle to enter the vein and catheterize it to thereby restore the blood channel and restore the blood flow. The needle and the tube are removed from the vein and then from the eye. An additional external surgery (sheathotomy, for example) can be associated with this procedure.

Needle 4 is made of a rigid material, such as a rigid plastic, stainless steel, etc. In a preferred embodiment, needle 4 may have length of 400-1500 microns, preferably 500 microns; and may have an external diameter of 30-120 microns, preferably 60 microns. The distal end 2d of the handpiece 2 may have an outer diameter of 0.5 mm to 2.5 mm, preferably about 1.0 mm; a length of 1.0-2.0 mm, preferably about 1.5 mm, before the bend "X"; and a length of 35-50 mm, preferably about 40 mm, between the bend "X" and the finger-grip 2a. As indicated earlier, the angle "α" between the longitudinal axes LA₁ of the needle 4 and LA₂ of the proximal end 2p of the handpiece 2 should be from 90°-180°, preferably from 120°-170°, most preferably about 145°. Such a construction facilitates penetration of the retinal plane closer to the central retinal vein to treat the occlusion.

During the foregoing procedures endoillumination is provided by the optical fiber 14 from the light source 16.

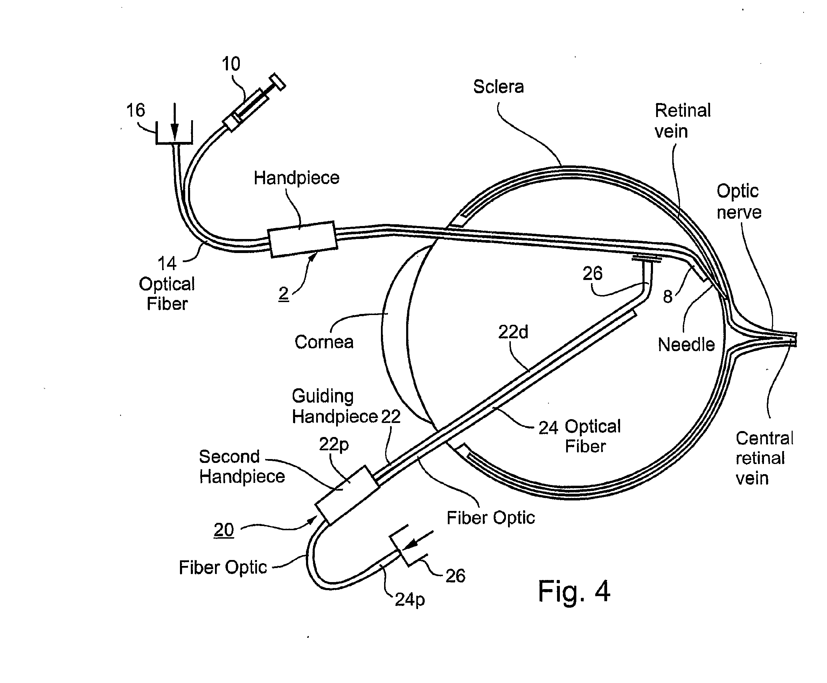

Fig. 4 illustrates an embodiment of the invention wherein the endoillumination is provided partly or completely by a separate microsurgical illumination instrument, generally designated 20 in Fig. 4. Fig. 4 illustrates the injection instrument described above in Figs. 1 - 3 as also including the illuminating optical fiber 14 for illuminating the injection site. It will be appreciated, however, that when using the illumination instrument 20 shown in Fig. 4, the optical fiber 14 in the injection instrument may be omitted, or may be included in order to provide more illumination at the injection site.

The illumination instrument 20 shown in Fig. 4 also includes a handpiece 22 having a proximal end 22p graspable by the physician, and a distal end 22d to be inserted into the subject's eye. In this case, however, handpiece 22 is formed with a single longitudinal passageway for receiving only an optical fiber 24. Optical fiber 24 has a proximal end 24p exposed to a light source 26, and a distal end 24d entering the subject's eye during the operation and oriented so as to illuminate the needle 4 of the injection instrument, and the injection site in the subject's retina.

The distal end 22d of the handpiece 22 also includes an extension 26 engagable with the distal end 2d of the injection instrument handpiece 2 within the subject's eye, adjacent to the injection site, for stabilizing and guiding the distal end of the injection handpiece when inserted into the subject's eye.

In all other respects, the procedure using the illumination instrument 20, together with the injection instrument described above with respect to Figs 1-3, may be the same as described above.

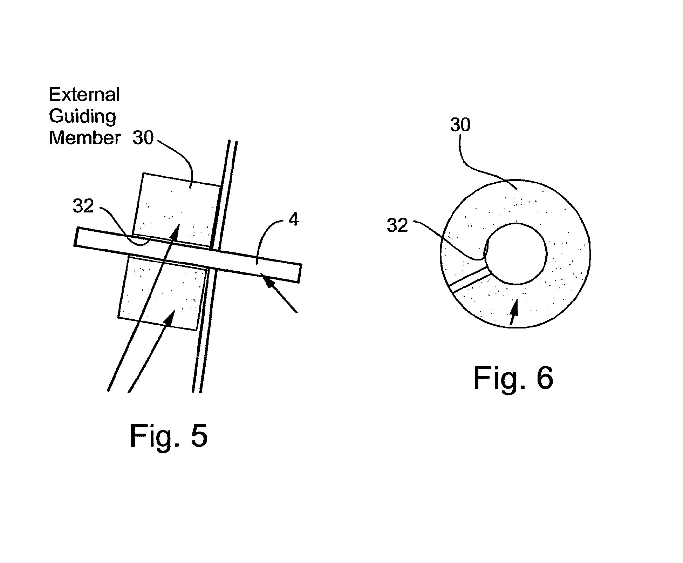

Figs. 5 and 6 illustrate the provision of an external guiding member, generally designated 30, for placement against the outer surface of the eye (sclera), and formed with a hole 32 for receiving and guiding the hollow needle 4 to penetrate the blood vessel in the subject's retina. Preferably, the external guiding member 30 is of a hollow cylindrical configuration and is made of a soft material, such as soft plastic, which controls the guided movement of the hollow needle during the insertion operation. Such a guiding member stabilizes the hollow needle when inserted, so that when the fibrinolytic injection is performed, the needle is stable in the retinal vein. Guiding 30 also facilitates the removal of the needle from the eye.

While the invention has been described with respect to several preferred embodiments, it will be appreciated that these are set forth merely for purposes of example, and that many variations may be made. For example, the described instrument could also be used for injecting a coagulant or other medication into the eye. Other variations and applications of the invention will be apparent.

## Claims

1. A microsurgical instrument for penetrating a blood vessel in a subject's eye, comprising:
a handpiece (2) having a proximal end (2p) graspable by the physician, and a distal end (2d) carrying a hollow needle sharpened at its tip, and
said handpiece being formed with at least one passageway (6) therethrough from its proximal end (2p) to said hollow needle (4) at the distal end (2d), and a longitudinal axis (LA₁) of the hollow needle (4) being at an angle between 90°-180° to the longitudinal axis (LA₄) of the proximal end (2p) of the handpiece (2), to facilitate orienting said needle (4) substantially tangentially to the plane of the subject's retina and thereby to facilitate penetrating the blood vessel in the subject's retina, **characterized in that** said angle can be varied.

2. The instrument according to Claim 1, wherein said handpiece includes a second passageway (12) therethrough from its proximal end (2p) to its distal end (2d); and an optical fiber (14) in said second passageway (12) for illuminating the hollow needle (4) and the blood vessel to be penetrated by the hollow needle (4).

3. The microsurgical instrument according to Claim 1, wherein the longitudinal axis of the distal end (2d) of the handpiece (2) and of the hollow needle (4) are at an angle of from 90°-180° to the longitudinal axis of the proximal end (2p) of the handpiece (2), to facilitate orienting said needle substantially tangentially to the plane of the subject's retina and thereby to facilitate its penetrating the blood vessel in the subject's retina, and
wherein said microsurgical instrument further comprising a stabilizing means (40) associated with a distal portion of said hand-piece (2), and wherein said stabilizer (40) being positionable against the retina and serves for stabilization while penetrating the blood vessel in the subject's retina.

4. The instrument according to Claim 1, wherein said hollow needle (4) has an outer diameter of 50 - 200 microns.

5. The instrument according to Claim 1, wherein said hollow needle (4) has a length of approximately 400 - 2000 microns.

6. The instrument according to Claim 1, wherein said angle between the longitudinal axes of the distal (2d) and proximal ends (2p) of the handpiece (2) is approximately 145°.

7. The instrument according to Claim 1, wherein the instrument further comprises a syringe (10) at the proximal end (2p) of the handpiece (2) communicating with said distal end of the needle (4) for feeding therethrough a liquid substance to be injected.

8. The instrument according to Claim 1, wherein the instrument further comprises a tube extended through said hollow needle (4) for catheterizing blood vessels.

9. A microsurgical injection instrument according to Claim 1, comprising also a microsurgical illumination instrument (20) for use therewith;
said microsurgical illumination instrument (20) also comprising a handpiece 22) having a proximal end (22p) graspable by the physician, and a distal end (22d) to be inserted into the subject's eye and also being formed with a passageway therethrough from said proximal end (22p) to said distal end (22d);
said latter passageway including an optical fiber having a proximal end to be exposed to a source of light, and a distal end to be located in the vicinity of the injection site in the subject's eye to illuminate same.

10. A surgical apparatus according to Claim 9, and wherein the distal end (22d) of said handpiece (22) of the microsurgical illumination instrument (20) is engagable with the distal end (2d) of the microsurgical injection instrument for stabilizing and guiding the distal end (2d) of said injection instrument in the subject's eye.

11. The instrument according to Claim 1, further comprising a stabilizing means (30) is external to the eye includes a hole for receiving and guiding said hollow needle (4) to penetrate the blood vessel in the subject's retina.

## Patentansprüche

1. Ein mikrochirurgisches Instrument zum Penetrieren eines Blutgefäßes in einem Auge einer Person bestehend aus:
einem Handteil (2) mit einem proximalen Ende (2p), das vom Arzt gehalten wird und einem distalen Ende (2d), das eine Hohlnadel (4) enthält, die am oberen Ende angespitzt ist und
das besagte Handteil formt mindestens einen Durchgang (6), welcher vom proximalen Ende (2p) zur besagten Hohlnadel (4) am distalen Ende (2d) und einer Längsachse (LA₁) der Hohlnadel (4) einen Winkel zwischen 90° - 180° zu der Längsachse (LA₄) des proximalen Endes (2p) des Handteils (2) hat, um die Orientierung der besagten Nadel (4) wesentlich tangential zur Fläche der Netzhaut der Person zu erleichtern und um **dadurch** die Penetration des Blutgefäßes der Person zu ermöglichen. Der besagte Winkel kann variieren.

2. Das Instrument entsprechend des Anspruchs 1, worin das besagte Handteil einen zweiten Durchgang (12) vom proximalen Ende (2p) zum distalen Ende (2d) hat und einen Lichtleiter (14) in dem besagten zweiten Durchgang (12) zum Beleuchten der Hohlnadel (4) und des Blutgefäßes, das mit der Hohlnadel (4) penetriert werden soll.

3. Das mikrochirurgische Instrument entsprechend des Anspruchs 1, worin die Längsachse des distalen Endes (2d) des Handteils (2) und von der Hohlnadel (4) einen Winkel von 90° - 180 zu der Längsachse des proximalen Endes (2p) des Handteils (2) haben, um die Orientierung der besagten Nadel tangential zur Fläche der Netzhaut der Person zu ermöglichen und **dadurch** das Penetrieren des Blutgefäßes in der Netzhaut der Person zu ermöglichen und
worin besagtes mikrochirurgisches Instrument stabilisierende Mittel (40) einschließt, welche mit dem distalen Teil des besagten Handteils (2) verbunden sind und besagter Stabilisator (40) gegen die Netzhaut positioniert werden kann. Dieser dient während der Penetration des Blutgefäßes in die Netzhaut der Person zur Stabilisierung.

4. Das Instrument entsprechend des Anspruchs 1, worin die besagte Hohlnadel (4) einen Außendurchmesser von 50-200 Mikronen hat.

5. Das Instrument entsprechend des Anspruchs 1, worin die besagte Hohlnadel (4) eine Länge von ungefähr 400-2000 Mikronen hat.

6. Das Instrument entsprechend des Anspruchs 1, worin besagter Winkel zwischen der Längsachse des distalen (2d) und des proximalen Endes (2) des Handteils (2) ungefähr 145° beträgt.

7. Das Instrument entsprechend des Anspruchs 1, worin das Instrument ferner über eine Spritze (10) am proximalen Ende (2p) des Handteils (2) verfügt, welche mit dem besagten distalen Ende der Nadel (4) zusammenarbeitet, um die Flüssigkeit aufzunehmen, die injiziert werden soll.

8. Das Instrument entsprechend des Anspruchs 1, worin das Instrument ferner aus einem Röhrchen besteht, welches eine Verlängerung der Hohlnadel (4) ist und zur Katheterisierung der Blutgefäße verwendet wird.

9. Ein mikrochirurgisches Injektionsinstrument entsprechend des Anspruchs 1, ebenfalls bestehend aus einem mikrochirurgischen Beleuchtungsinstrument (20) zur Verwendung mit diesem.
Das besagte mikrochirurgische Beleuchtungsinstrument (20) besteht ebenfalls aus einem Handteil (22), das ein proximales Ende (22p) hat, das der Arzt hält und ein distales Ende (22d), das in das Auge der Person eingeführt wird, das auch über einen Durchgang verfügt, der vom besagten proximalen Ende (22p) zum besagten distalen Ende (22d) reicht.
Der letztere Durchgang umfaßt einen Lichtleiter mit einem proximalen Ende, welcher einer Lichtquelle ausgesetzt wird und ein distales Ende, das sich in der Nähe der Injektionsstelle des Auges der Person befinden sollte, um dieses zu beleuchten.

10. Ein chirurgisches Gerät entsprechend des Anspruchs 1, worin das distale Ende (22d) des besagten Handteils (22) des mikrochirurgischen Instruments (20) mit dem distalen Ende (2d) des Injektionsinstruments verbunden werden kann, um das distale Ende des besagten Injektionsinstruments im Auge der Person zu stabilisieren und zu führen.

11. Das Instrument entsprechend des Anspruchs 1, das ferner aus einem Stabilisator (30) besteht, liegt extern zum Auge und umfasst ein Loch, um die Hohlnadel (4) aufzunehmen und zu führen und um das Blutgefäß in der Netzhaut der Person zu penetrieren.

## Revendications

1. Un instrument micro-chirurgical permettant de pénétrer dans le vaisseau sanguin de l'oeil d'un sujet, comprenant:
Un manche (2) doté d'une extrémité proximale (2p) que peut saisir le médecin, et d'une extrémité distale (2d) équipée d'une aiguille creuse à bout pointu, et
le manche en question comprenant au moins un passage (6) allant de son extrémité proximale (2p) vers l'aiguille creuse mentionnée (4) à l'extrémité distale (2d), et un axe longitudinal (LA1) de l'aiguille creuse (4) faisant un angle de 90° à 180° avec l'axe longitudinal (LA4) de l'extrémité proximale (2p) du manche (2), afin de faciliter l'orientation de l'aiguille (4) de manière substantielle et tangente au plan de la rétine du sujet et afin de faciliter ainsi la pénétration dans le vaisseau sanguin de la rétine du sujet, qui se **caractérise par le fait que** cet angle peut varier.

2. L'instrument selon la revendication 1, dans lequel le manche mentionné inclue un second passage (12) depuis son extrémité proximale (2p) vers son extrémité distale (2d); et une fibre optique (14) dans ce second passage (12) pour éclairer l'aiguille creuse (4) et le vaisseau sanguin dans lequel doit pénétrer l'aiguille creuse (4).

3. L'instrument micro-chirurgical selon la revendication 1, dans lequel l'axe longitudinal de l'extrémité distale (2d) du manche (2) et de l'aiguille creuse (4) forment un angle de 90° à 180° par rapport à l'axe longitudinal de l'extrémité proximale (2p) du manche (2) afin de faciliter l'orientation de l'aiguille de manière substantielle et tangente au plan de la rétine du sujet, ce qui permet de pénétrer plus facilement dans le vaisseau sanguin de la rétine du sujet, et
dans lequel cet instrument micro-chirurgical comprend en outre un moyen de stabilisation associé à une portion distale du manche mentionné (2), ce moyen de stabilisation pouvant être placé contre la rétine et servir de stabilisateur lorsqu'on pénètre dans le vaisseau sanguin de la rétine du sujet.

4. L'instrument selon la revendication 1, dans lequel l'aiguille creuse mentionnée (4) a un diamètre extérieur de 50-200 microns.

5. L'instrument selon la revendication 1, dans lequel l'aiguille creuse mentionnée (4).a une longueur d'environ 400-2000 microns.

6. L'instrument selon la revendication 1, dans lequel l'angle mentionné entre les axes longitudinaux des extrémités distale (2d) et proximale (2p) du manche (2) est d'environ 145°.

7. L'instrument selon la revendication 1, dans lequel l'instrument comprend en outre une seringue (10) à l'extrémité proximale (2p) du manche (2) communiquant avec l'extrémité distale (2d) mentionnée de l'aiguille (4) afin d'y introduire une substance liquide à injecter.

8. L'instrument selon la revendication 1, dans lequel l'instrument comprend en outre un tube introduit dans ladite aiguille creuse (4) pour un cathétérisme des vaisseaux sanguins.

9. Un instrument d'injection micro-chirurgical selon la revendication 1, comprenant aussi un dispositif d'éclairage micro-chirurgical (20) à utiliser avec celui-ci; ledit dispositif d'éclairage micro-chirurgical (20) comprenant également un manche (22) ayant une extrémité proximale (22p) que le médecin peut saisir, et une extrémité distale (22d) à insérer dans l'oeil du sujet, et comprenant aussi un passage allant de l'extrémité proximale (22p) à l'extrémité distale (22d);
ledit passage comprenant une fibre optique ayant une extrémité proximale devant être exposée à une source lumineuse, et une extrémité distale devant être placée à proximité du site d'injection dans l'oeil du sujet, afin de l'éclairer.

10. Un appareillage chirurgical selon la revendication 9, dans lequel l'extrémité distale (22d) du manche (22) du dispositif d'éclairage micro-chirurgical peut s'émboîter dans l'extrémité distale (2d) de l'instrument d'injection micro-chirurgical afin de stabiliser et de guider l'extrémité distale (2d) de cet instrument d'injection dans l'oeil du sujet.

11. L'instrument selon la revendication 1, comprenant en outre un moyen de stabilisation (30), extérieur à l'oeil et incluant un trou pour recevoir et guider ladite aiguille creuse (4) afin qu'elle pénètre dans le vaisseau sanguin de la rétine du sujet.
